Europäisches Patentamt

European Patent Office

Office européen des brevets

(19)

(11) **EP 1 007 962 B1**

(12) **FASCICULE DE BREVET EUROPEEN**

(45) Date de publication et mention
de la délivrance du brevet:
**29.01.2003 Bulletin 2003/05**

(21) Numéro de dépôt: **98942827.1**

(22) Date de dépôt: **04.09.1998**

(51) Int Cl.$^7$: **G01N 33/00**

(86) Numéro de dépôt international:
**PCT/FR98/01897**

(87) Numéro de publication internationale:
**WO 99/012029 (11.03.1999 Gazette 1999/10)**

(54) **APPAREIL ET METHODE DE CLASSIFICATION UTILISANT UNE COMBINAISON DE METHODES STATISTIQUES ET DE RESEAUX NEURONAUX**

KLASSIFIZIERANLAGE UND KLASSIFIZIERVERFAHREN UNTER VERWENDUNG EINER KOMBINATION VON WARSCHEINLICHKEITSVERFAHREN UND NEURONALEN NETZWERKEN

CLASSIFYING APPARATUS AND METHOD USING A COMBINATION OF STATISTICAL METHODS AND NEURONAL NETWORKS

(84) Etats contractants désignés:
**DE FR GB**

(30) Priorité: **04.09.1997 FR 9711001**

(43) Date de publication de la demande:
**14.06.2000 Bulletin 2000/24**

(73) Titulaire: **ALPHA M.O.S.**
**31400 Toulouse (FR)**

(72) Inventeurs:
• **LABRECHE, Said**
**F-31200 Toulouse (FR)**
• **AMINE, Hicham**
**F-31400 Balma (FR)**
• **TAN, Tze, Tsung**
**F-31500 Toulouse (FR)**
• **LOUBET, François**
**F-31130 Balma (FR)**

(74) Mandataire: **Joly, Jean-Jacques et al**
**Cabinet Beau de Loménie**
**158, rue de l'Université**
**75340 Paris Cédex 07 (FR)**

(56) Documents cités:
**EP-A- 0 632 268        EP-A- 0 733 880**
**US-A- 5 373 452**

• **CORCORAN P: "ELECTRONIC ODOUR SENSING SYSTEMS" ELECTRONICS AND COMMUNICATION ENGINEERING JOURNAL, vol. 5, no. 5, 1 octobre 1993, pages 303-308, XP000435448**
• **CHASTRETTE M ET AL: "Tetralin, indan and nitrobenzene compound structure-musk odor relationship using neural networks" EUROPEAN JOURNAL OF MEDICINAL CHEMISTRY.CHIMICA THERAPEUTICA, vol. 30, no. 9, 1995, page 679-686 XP004040195**

EP 1 007 962 B1

**Description**

La présente invention concerne la classification de motifs, notamment dans le domaine de la caractérisation ou de l'identification d'odeurs.

Dans les appareils de caractérisation ou d'identification d'odeurs (dits "nez électroniques") un échantillon d'un produit inconnu est identifié en le classant dans une parmi plusieurs classes que l'appareil reconnaît grâce à une opération d'apprentissage préalable. Les appareils de ce genre comportent deux parties principales, une partie d'acquisition de données et une partie de traitement des données. La partie d'acquisition de données comporte, en général, une tête de prélèvement permettant de prélever des composants volatiles qui se dégagent du produit testé, et une batterie de capteurs auxquels sont fournis les composants prélevés et qui produisent des signaux de sortie qui, pris en combinaison, sont caractéristiques des composants volatiles dégagés par le produit et donc sont caractéristiques du produit. La partie de traitement de données comporte, en général, des moyens informatiques qui enregistrent et effectuent des analyses sur les signaux de sortie des capteurs. En décelant ou analysant les motifs inhérents aux différents ensembles de données relatif à des produits particuliers la partie de traitement de données détecte la classe du produit (qui peut correspondre soit à la nature du produit, par exemple, "un vin"; "un whisky"; etc., soit à une qualité du produit, par exemple, "lait frais"; "lait fermenté"; etc.).

Les objectifs poursuivis en acquérant et en traitant les données sont différents selon que l'appareil est en mode d'apprentissage ou en mode d'identification.

Dans la phase d'apprentissage de l'appareil, les produits dont sont extraits les différents échantillons sont connus et analysés plusieurs fois. Les méthodes de traitement utilisées sont alors celles qui permettent d'une part de détecter et caractériser les différents produits et d'autre part de construire un modèle robuste d'identification. Les méthodes utilisées sont généralement des méthodes statistiques (Analyse en Composantes Principales (ACP), Analyse Factorielle Discriminante (AFD),...) et des réseaux de neurones (voir par exemple, J.W. Gardner et P.N. Bartlett," Sensors and sensory systems form an Electronic nose", Nato Asi, Series E Vol. 212, pp. 161-180 (1992)). Dans la phase d'identification, l'origine des échantillons n'est pas connue et l'objectif est d'identifier cette origine en utilisant les données et le modèle déjà construit.

Les méthodes statistiques utilisées dans le traitement des données enregistrées par un nez électronique sont des méthodes linéaires. Les variables calculées selon de telles méthodes sont dites "variables synthétiques" et portent deux informations, une information quantitative, indiquant le pourcentage d'inertie du nuage initiale expliqué par cette variable, et une information qualitative quant à la structure et la discrimination du nuage qu'elle révèle. Par contre, les réseaux de neurones font partie des méthodes non linéaires et sont capables d'apprendre le comportement des différents capteurs. Les deux types de méthodes utilisent directement les données brutes enregistrées. Elles sont en général utilisées séparément.

Par ailleurs, il est rappelé que l'expression "variables synthétiques" se réfère à des combinaisons (linéaires ou non) des variables mesurées. Elles sont généralement obtenues via l'optimisation d'un critère bien défini. L'environnement de cette optimisation peut être conditionné par des connaissances ou des variables supplémentaires. Par exemple, en ACP on optimise un critère sans information supplémentaire tandis qu'en AFD on a la connaissance de l'appartenance de chaque échantillon à un groupe, et en PLS (en anglais "Partial Least Square") une ou plusieurs variables supplémentaires sont mesurées sur chaque échantillon (par exemple la concentration d'un élément chimique particulier).

Le document WO96/26492 décrit une structure de réseau neuronal particulier susceptible, entre autres, d'être utilisé dans les nez électroniques. Ce réseau neuronal comporte dans sa couche de sortie des neurones de logique floue permettant de signaler que le produit testé appartient à une classe inconnue. Dans ce document, on propose également d'augmenter le nombre de neurones dans la couche d'entrée d'un réseau de neurones afin d'appliquer en entrée, non seulement les signaux de sortie des capteurs, mais aussi des signaux représentant des variables calculées dans une Analyse en Composantes Principales des signaux originaux, par exemple les deux ou trois premières composantes de l'ACP.

Cependant, en ajoutant de façon brute les signaux résultant d'une Analyse en Composantes Principales des données aux signaux mesurés représentant les données pour constituer les signaux d'entrée au réseau neuronal, WO96/26492 reste dans la lignée des procédés classiques qui cumulent les procédés statistiques et les procédés neuronaux. De plus, même si les variables obtenues par une analyse en composantes principales apportent des renseignements quantitatives importantes, leur apport en renseignements qualitatives, particulièrement en cas de traitement d'un grand nombre de produits distincts, n'est pas nécessairement grand. Alors, l'utilisation systématique d'un certain nombre de variables prédéterminées issues d'une analyse statistique n'assure pas automatiquement la discrimination des différents produits. De plus, ces variables sont corrélées avec certaines des variables (canaux) initiales et, par conséquent, leur utilisation en tant que signaux d'entrée au réseau neuronal prêt à la redondance.

La présente invention à pour objet un appareil de classification de motifs, notamment destiné à une utilisation dans le domaine de la reconnaissance d'odeurs, dans lequel les procédés statistiques et les procédés neuronaux sont imbriqués afin d'utiliser au maximum la synergie entre eux, et un procédé d'optimisation d'un tel appareil pendant une phase d'apprentissage de celui-ci.

Plus particulièrement, la présente invention prévoit un appareil de classification, notamment destiné à une utilisation dans la reconnaissance ou la caractérisation d'odeurs, comprenant: un moyen destiné à acquérir, pendant une phase d'apprentissage et par une pluralité de canaux, des données brutes représentant une pluralité d'instances d'une pluralité de classes différentes ; une unité de traitement destinée à traiter lesdites données brutes afin de déterminer, pour chaque classe (j), des caractéristiques communes aux différentes instances de la classe; et un réseau neuronal destiné à émettre en sortie une classification dans une des classes d'une instance présentée audit appareil de classification, ledit réseau neuronal comportant une couche d'entrée, une couche de sortie et au moins une couche intermédiaire disposée entre les couches d'entrée et de sortie, la couche de sortie comprenant une pluralité de neurones chacun étant adapté à reconnaître des instances d'une classe respective de la pluralité de classes ; caractérisé en ce que l'unité de traitement détermine pour chaque classe (j) un sous-espace (SEj) dans lequel des instances de cette classe (j), pour lesquelles des données brutes ont été obtenues, sont séparées de façon optimale des instances de toutes les autres classes, ledit sous-espace (SEj) étant défini par un ensemble (VDj) d'une ou plusieurs variables synthétiques (V), et de déterminer le sous-espace de discrimination (SED) qui comprend les sous-espaces (SEj) de toutes les classes, ledit sous-espace (SED) étant défini par la pluralité de variables (VD) comprises dans les ensembles (VDj) de variables synthétiques de la pluralité de classes ; et que la couche d'entrée du réseau neuronal comporte une pluralité de neurones correspondant chacun à une variable respective (V) de la pluralité de variables synthétiques (VD) qui définissent le sous-espace de discrimination (SED).

La présente invention prévoit, également, un procédé d'apprentissage d'un appareil de classification comprenant un moyen destiné à acquérir, par une pluralité de canaux, des données brutes représentant des instances d'une pluralité de classes différentes, une unité de traitement de ces données brutes et un réseau neuronal comportant une couche d'entrée, une couche de sortie et au moins une couche intermédiaire disposée entre les couches d'entrée et de sortie, la couche de sortie comprenant une pluralité de neurones chacun étant adapté à reconnaître des instances d'une classe respective de la pluralité des classes, cet appareil étant notamment destiné à une utilisation dans la reconnaissance ou la caractérisation d'odeurs, le procédé comportant les étapes consistant à : soumettre au moyen d'acquisition de données brutes, pendant une phase d'apprentissage, une pluralité d'instances de la pluralité de classes, l'appartenance des instances aux classes étant connue ; et traiter lesdites données brutes, par l'unité de traitement de données afin de déterminer pour chaque classe (j), des caractéristiques communes aux différentes instances de la classe; caractérisé en ce que l'étape de traitement comporte la détermination par l'unité de traitement de données, pour chaque classe (j), d'un sous-espace (SEj) dans lequel des instances de cette classe (j), pour lesquelles des données brutes ont été obtenues, sont séparées de façon optimale des instances de toutes les autres classes, ledit sous-espace (SEj) étant défini par un ensemble (VDj) d'une ou plusieurs variables synthétiques (V), et de déterminer le sous-espace de discrimination (SED) qui comprend les sous-espaces (SEj) de toutes les classes, ledit sous-espace (SED) étant défini par la pluralité de variables (VD) comprises dans les ensembles (VDj) de variables synthétiques de la pluralité de classes ; et que le réseau neuronal est muni d'une couche d'entrée comportant une pluralité de neurones destinés à recevoir, respectivement, en entrée la valeur d'une variable respective de la pluralité de variables synthétiques (VD) qui définissent le sous-espace de discrimination (SED).

L'appareil et le procédé de la présente invention utilisent l'imbrication des résultats d'une analyse statistique avec un réseau neuronal afin de bénéficier au maximum des avantages fournis par chacun de ces derniers. Plus particulièrement, en choisissant pour introduction dans le réseau neuronal les variables synthétiques VD qui discriminent au mieux les différentes classes, il y a une augmentation importante de l'apport en informations qualitatives ce qui mène à une réduction de la durée de la phase d'apprentissage et à une augmentation de la vitesse d'identification des échantillons au cours de la phase d'identification.

Le choix des variables synthétiques qui discriminent au mieux les différentes classes peut se faire manuellement ou selon une exploration automatique. Une méthode d'exploration automatique préférée consiste en la détermination, dans chacun d'une pluralité de sous-espaces différents correspondants à des combinaisons de variables différentes respectives, d'une région englobant les points représentant les différentes instances de cette classe et la détermination du sous-espace dans lequel la région déterminée est à la fois la plus éloignée des régions représentant les autres classes et la moins étendue possible.

Dans un mode de réalisation préféré de l'invention, le réseau neuronal comporté, en tant que couche intermédiaire, une pluralité de réseaux neuronaux individuels correspondants à une classe respective de la pluralité de classes. En utilisant cette structure du réseau neuronal, il est possible de réduire la durée de la phase d'apprentissage de manière importante, ce qui peut être d'une importance capitale lorsqu'il s'agit de la classification d'instances d'un grand nombre de classes. L'entraînement d'un tel réseau neuronal passe par l'entraînement individuel des réseaux neuronaux individuels.

Dans certaines applications il peut s'avérer utile, ou bien nécessaire, d'éliminer des analyses les données brutes relatives aux canaux qui ne contribuent pas à différencier les instances de classes différentes. Il peut aussi s'avérer nécessaire d'éliminer des calculs les données concernant des instances aberrantes d'une classe, c'est-à-dire, des instances qui, en termes de variables synthétiques, sont éloignées des autres instances de la même classe. Ceci augmente la fiabilité du modèle d'identification établi pendant la phase d'apprentissage.

De préférence les réseaux neuronaux de l'invention sont des réseaux supervisés dont les neurones mettent en oeuvre des fonctions sigmoïdes. De préférence, les réseaux neuronaux selon l'invention sont entraînés en employant l'algorithme de propagation vers l'avant.

L'unité de traitement des données brutes met en oeuvre des traitements statistiques tels qu'une analyse en composantes principales, une analyse factorielle discriminante, ou d'autres procédés similaires.

Afin de vérifier la fiabilité du modèle d'identification établit au cours de la phase d'apprentissage de l'appareil, il est préférable de déterminer les sous-espaces (SEj), les ensembles de variables synthétiques et le sous-espace de discrimination (SED) sur la base des données brutes relatives à un pourcentage seulement des données acquises pendant la phase d'apprentissage. Les autres données sont soumises à l'unité de traitement afin de calculer les valeurs correspondantes des variables synthétiques, ces valeurs sont soumises au réseau neuronal et les classifications effectuées par le réseau neuronal sont comparées aux véritables classes auxquelles appartiennent les instances concernées. Si le taux d'identification des classes dépasse une valeur de seuil, par exemple 80%, alors l'apprentissage de l'appareil est terminé et l'appareil peut être employé pour identifier des instances dont la classe est inconnue.

On va maintenant décrire des modes de réalisation particuliers de la présente invention, à titre d'exemples non limitatifs, en liaison avec les dessins annexés dans lesquels:

la figure 1 est un organigramme illustrant les différentes étapes des prétraitements statistiques qui sont appliqués dans le premier mode de réalisation préféré de l'invention ;
les figures 2A et 2B sont des graphiques montrant la distribution de données obtenues par une analyse en composantes principales et représentant des instances de cinq classes différentes dans un plan qui permet de différencier les différentes classes les unes des autres, où:

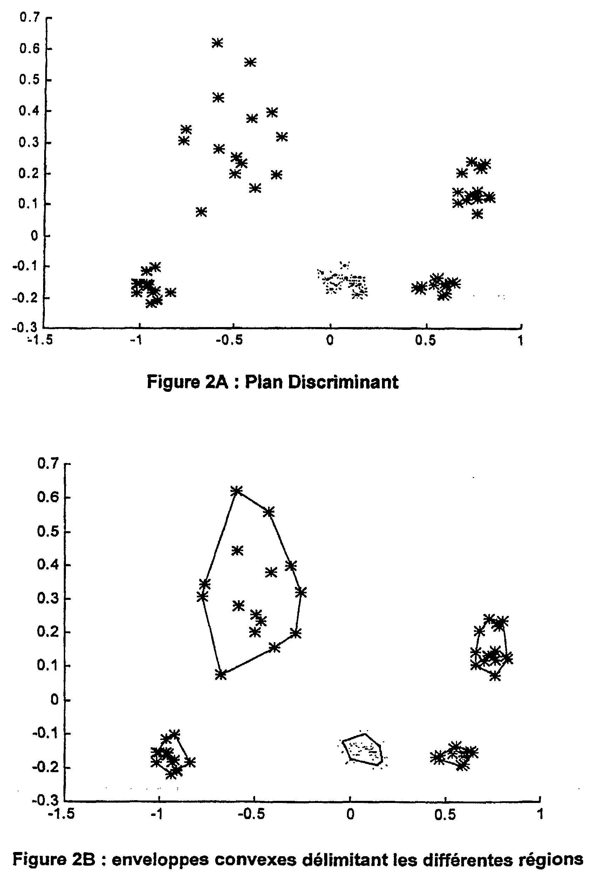

la figure 2A montre la distribution des points ; et
la figure 2B montre des frontières définies pour délimiter des régions correspondant aux classes différentes ;

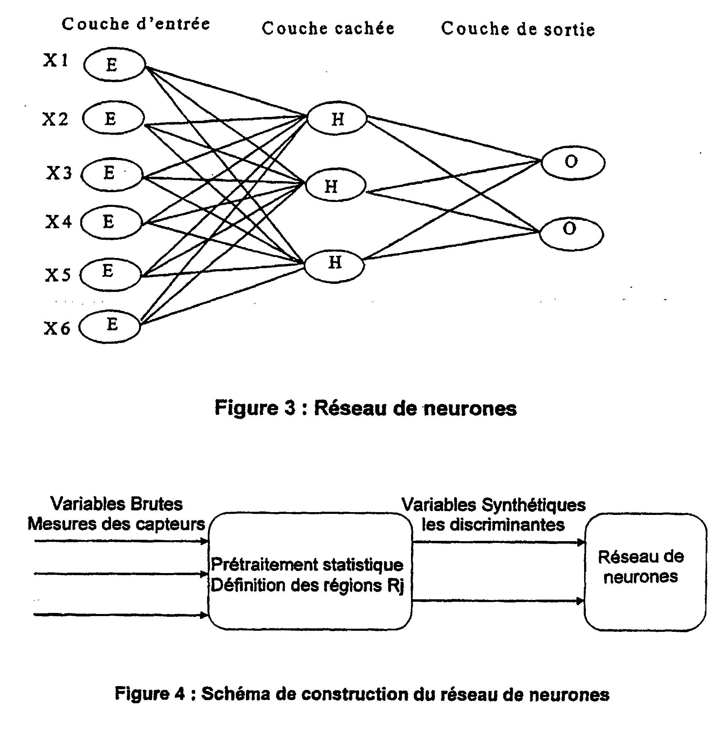

la figure 3 est un schéma indiquant la structure globale d'un réseau neuronal classique ;
la figure 4 est un schéma illustrant les étapes principales dans l'établissement d'un réseau neuronal global selon le premier mode de réalisation préféré de l'invention;

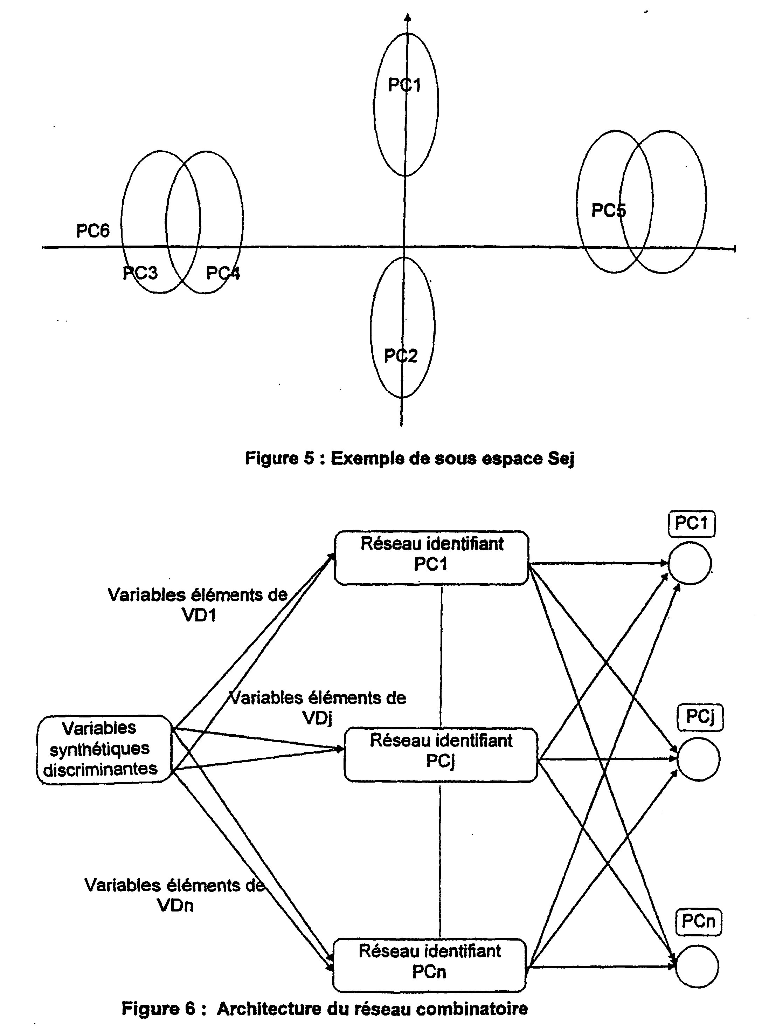

la figure 5 est un graphique illustrant le cas d'un sous-espace dans lequel deux classes parmi six classes de produits sont bien différenciées des autres ; et
la figure 6 illustre de façon schématique la structure d'un réseau neuronal selon le deuxième mode de réalisation de l'invention.

Des modes de réalisation préférés de l'appareil selon la présente invention seront maintenant décrits dans le contexte d'appareils de reconnaissance d'odeurs. Il est à comprendre, cependant, que la présente invention n'est pas limitée à de telles applications mais s'applique aussi bien à d'autre domaines où des données représentant des instances de classes différentes doivent être classées.

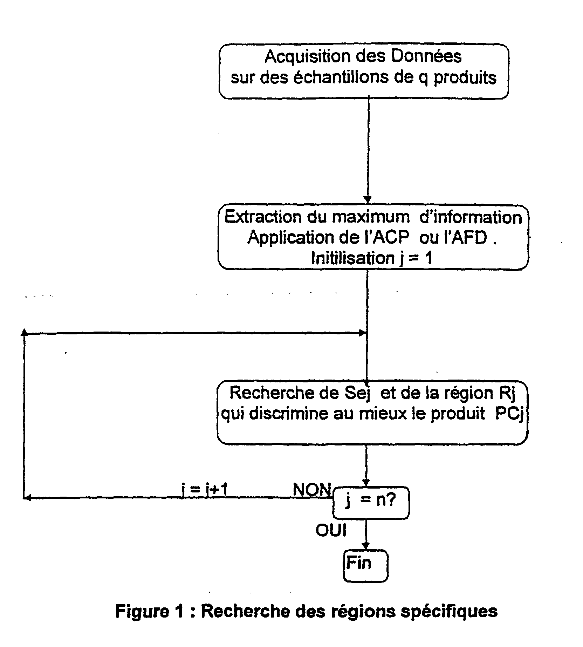

Un premier mode de réalisation de la présente invention sera maintenant décrit à l'aide des figures 1 à 4. Ce mode de réalisation utilise un appareil du type dit "nez électronique" comportant p capteurs. Ces capteurs peuvent inclure des capteurs de types classiques tels, par exemple, des capteurs à polymère conducteur, des capteurs piézo-électriques à quartz; des capteurs à oxyde semiconducteur, etc. Au cours de la phase d'apprentissage de l'appareil, plusieurs échantillons de produits connus sont présentés à l'appareil afin de générer des données brutes (une base d'apprentissage) qui seront ensuite analysées pour établir un modèle d'identification. L'analyse consiste en la réalisation de certains prétraitements statistiques, l'affectation à chaque classe des variables synthétiques permettant de discriminer au mieux les instances de cette classe des instances des autres classes, et l'établissement de la structure d'un réseau neuronal sur la base des résultats de ces procédés. La figure 1 résume les différentes étapes de cette analyse.

## PRETRAITEMENTS STATISTIQUES

### L'établissement d'une base d'apprentissage

Plusieurs capteurs sélectionnés sont utilisés dans un "nez électronique". A chaque échantillon analysé i, on associe un point $\chi_i$ dans un espace de dimension **p, p** étant le nombre de capteurs.

$$\chi_i = \sum_{k=1}^{p} x(i,k)\, e_k \qquad \text{avec } k = 1 \text{ à } p$$

$e_k$ est un vecteur de dimension $p$ dont les composantes sont toutes égales à 0 sauf le $k^{\text{ème}}$ qui vaut 1. L'ensemble de ces vecteurs forme une base de l'espace. $x(i,k)$ est la mesure donnée par la $k^{\text{ème}}$ capteur lors de l'analyse de $i$. L'ensemble de ces représentations forme un nuage de points.

Généralement, lors de l'apprentissage de cet appareil, plusieurs échantillons sont analysés. Selon les applications, ces échantillons peuvent être extraits de différents produits (**PCJ** /$j$ = 1 à $n$) ou d'un même produit à différents états (bon, mauvais, marginal) ou à différentes concentrations. A la fin de la première étape de la phase d'apprentissage (la phase "acquisition de données"), on obtient donc un tableau ou matrice $X$ de données de dimensions $(m,p)$, $m$ étant le nombre total d'échantillons analysés (c'est-à-dire que $i$ prend des valeurs comprises entre 1 à $m$).

Le but recherché est alors de déterminer si l'appareil permet :

- de discriminer les différents produits,
- d'identifier les échantillons d'un même produit.

Sur l'espace contenant les points, on défini une métrique $M$. On défini aussi certains éléments statistiques sur le tableau $X$ centré, en particulier les matrices des covariances $V$, des variances interclasse B, intraclasse W et les centres de gravités ($g_j$ / $j$ = {1;$n$}, $n$ étant le nombre de produits analysés) des classes définies par les échantillons des différents produits (voir, par exemple, G. Saporta "Probabilités, Analyse des données et Statistique", Editions TECHNIP (1990) pour le calcul de ces éléments).

**Choix de capteurs**

Au cours des prétraitements statistiques, les capteurs qui discriminent au mieux les différents produits sont choisis automatiquement. Ce choix est basé sur la recherche pas à pas de la meilleure combinaison. M.C. Costanza et AA Afifi ont proposé dans (Journal of the American Statistical Association, Vol. 74, Number 368 (1979)) un algorithme approprié. Les capteurs non choisis ne seront pas pris en compte dans toutes les analyses.

**Détermination des variables synthétiques**

Des variables synthétiques peuvent être affectées à chaque produit en employant des analyses statistiques de plusieurs genres, par exemple, l'ACP ou l'AFD.

L'ACP permet de remplacer les capteurs choisis par de nouvelles variables synthétiques ($Cj$, $j$ = {1;$p$}) non corrélées et qui expliquent le maximum de l'information quantitative contenue dans le tableau de données. En ACP, on cherche une base particulière ($cj$, $j$ = {1;$p$}) de l'espace. La variable synthétique $Cj$ est alors associée au vecteur $cj$.

Les vecteurs ($cj$,$j$ = {1;$p$}) sont les vecteurs propres $M$ orthonormés de la matrice $VM$. Voir, par exemple, Gene H. Golub & Charles F. Van Loan "Matrix Computations", The Johns Hopkins University Press (1990) pour le calcul des éléments propres. Les variables synthétiques sont alors définies par

$$Cj(x) = x'Mcj$$

avec $j$ = {1;$p$}, $x'$ étant la transposé de $x$. Généralement $M$ est associée à la matrice identité.

L'AFD pour sa part, permet de remplacer les capteurs choisis par de nouvelles variables synthétiques ($Uj$, $j$ = {1;$p$}) qui discriminent au mieux les différents produits. En AFD, on cherche une base particulière ($uj$, $j$ = {1;$p$}) de l'espace. La variable synthétique $Uj$ est alors associée au vecteur $uj$.

Les vecteurs ($uj$, $j$ = {1;$p$}) sont les vecteurs propres $W^{-1}$ orthonormés de la matrice $BW^{-1}$. Voir supra Gene H. Golub & Charles F. Van Loan "Matrix Computations" pour le calcul des éléments propres. Les variables synthétiques sont alors définies par

$$Uj(x) = x'W^{-1}uj$$

avec $j$ = {1;$p$}, $x'$ étant la transposée de $x$, et $W^{-1}$ l'inverse de la matrice $W$.

## AFFECTATION DES VARIABLES SYNTHETIQUES A CHAQUE CLASSE

L'exploration des différents sous espaces engendrés par les nouvelles variables synthétiques *V* (qu'elles soient déterminées par l'ACP ou par l'AFD ou par une autre méthode) permet de discriminer chacun des produits. Cette exploration peut se faire manuellement en visualisant les différents plans ou d'une manière automatique en cherchant la meilleure combinaison de ces variables. Ce procédé permet d'associer à chaque produit *PCj* l'ensemble *VDj* des variables synthétiques *V* qui le discriminent au mieux des autres produits. Cet ensemble *VDj* de variables engendrent un sous espace *SEj* de dimension *pj* dans lequel une région spécifique *Rj* lui sera associée. Cette région ne doit pas chevaucher celle d'un autre produit ou bien la région de chevauchement doit être aussi petite que possible. Le sous espace *SEj* est décrit par un nombre de variables synthétiques inférieur à *p.* L'ensemble de toutes ces variables synthétiques forment le sous espace de discrimination noté *SED.*

$$SED = \bigcup_{j=1}^{n} SEj$$

La figure 2A illustre un exemple des résultats d'une analyse typique et représente la projection des échantillons de cinq produits sur le plan défini par deux variables synthétiques obtenues par une ACP et identifiées par une exploration des différentes possibilités. On observe que les différents produits sont bien discriminés. Dans ce cas tous les sous espaces *SEj* et *SED* sont confondus avec ce plan unique, c'est-à-dire que pour toutes les classes *j* les variables synthétiques *VDj* qui permettent de les discriminer au mieux sont les mêmes ($VD_1 = VD_2 = VD_3 = VD_4 = VD_5$).

La recherche de VDj peut être effectué visuellement, en affichant des représentations graphiques des points de mesure dans des sous-espaces de deux ou trois dimensions (correspondantes à des combinaisons de deux ou trois variables V). Cette exploration peut aussi être réalisée automatiquement suivant, par exemple, le procédé décrit ci-dessous.

On note CV l'ensemble de toutes les combinaisons possibles des variables synthétiques. Pour chaque élément CV(k) de CV, on projette l'ensemble des points correspondants aux données brutes sur le sous espace *SEk* défini par les variables de la combinaison CV(k) concernée. Sur l'espace de projection, les enveloppes convexes de chaque classe sont calculées. Ces enveloppes convexes délimitent des régions *Rj* correspondant à chaque produit (classe), comme le montre la figure 2B pour l'exemple de la Figure 2A. Plusieurs algorithmes permettent de définir cette enveloppe. En particulier B. Chazelle dans ("Discrete Computational Geometry", 10, pp. 377-409 (1993)) a proposé un algorithme pour le cas multidimensionnel.

Si la surface ou le volume de la région *Rj* délimitée par l'enveloppe associée à la classe *j* dans le sous espace *SEk* est notée *CV(k,j),* et la surface ou le volume séparant deux classes *j'* et *j* dans ce sous-espace *SEk* est notée *CV(k,j'j)* (dont la valeur est 0 en cas de chevauchement des classes *j* et *j'*), il est préférable d'associé à la classe *j* la classe *kj* qui minimise *CV(k,j)* et pour qui *CV(k,j',j)* est maximale. *VDj* sera alors la combinaison de variables telle que

$$\frac{CV(k,kj,j)}{CV(k,j)}$$

est maximale. En d'autres termes, *VDj* définit le sous-espace *SEj* dans lequel la classe j est le mieux séparée de toutes les autres classes.

Dans le cas où pour chaque *k, CV(k,kj,j)* = 0, c'est-à-dire que la classe *j* chevauche avec la classe *kj* dans tous les sous-espaces possibles, alors le critère suivant peut être utilisé: choisir la combinaison de variables *VDj* pour laquelle $\frac{CV(k,j)}{CVi(k,kj,j)}$ est maximale, où *Cvi(k,j',j)* est une mesure de l'intersection des deux classes *j* et *j'*.

Les méthodes statistiques permettent aussi de détecter les points aberrants. Un échantillon d'un produit *PCj* sera identifié comme aberrant, si en éliminant ce point la variation du volume ou surface de la région *Rj* est supérieure à un certain pourcentage (par exemple 10%).

Les points aberrants détectés sont éliminés de la base de données. Leur élimination permet de filtrer la base. La méthode statistique utilisée est ensuite réappliquée sur la base filtrée.

Dans les modes de réalisation décrits ci-dessus, le choix des variables VDj est réalisé en cherchant à discriminer au mieux chaque classe des autres. Pourtant, on peut envisager le cas où deux combinaisons de variables $VDj^A$, $VDj^B$ permettent de différencier une classe *j* des autres et que la combinaison $VDj^A$ qui permet de mieux différencier cette classe de toutes les autres utilise une ou plusieurs variables *V* qui sont utiles uniquement par rapport à la discrimination de cette classe *j.*Dans un tel cas, afin de réduire au minimum le nombre total de variables nécessaires pour discriminer les différentes classes les unes des autres (et de réduire la dimension du sous espace de discrimination SED), on peut choisir d'associer la classe j à la combinaison de variables $VDj^B.$

Il convient de noter que, dans certaines applications, l'une ou plusieurs (voire toutes) des variables synthétiques qui discriminent au mieux les différentes classes peuvent être constituées, respectivement, d'une ou de plusieurs des variables initiales, c'est-à-dire les signaux de sortie d'un des capteurs. Plus particulièrement, une variable synthétique V (qu'on va appeler ici $Y^k$) peut être constituée d'une combinaison linéaire des variables initiales X et, donc, en notant l'ensemble des variables initiales $X^j$, où j = 1 à p,

$$Y^k = \sum_{j=1}^{p} \alpha_{kj} X^j$$

(les coefficients $\alpha_{kj}$ sont des réels). On peut, donc, associer à une variable synthétique de ce genre une variable $X^k$ d'un sous-ensemble des variables initiales qui discriminent au mieux une classe des autres, où

$$X^k = \sum_{j=1}^{p} \alpha_{kj} X^j$$

Or, dans certains cas le processus d'optimisation des critères de discrimination (tels que ceux décrits ci-dessus), résultera en une détermination que, pour une ou plusieurs des variables synthétiques de ce genre, tous les coefficients $\alpha_{kj}$ seront égaux à zéro, sauf $\alpha_{kk}$ qui vaudra 1.

## CONSTRUCTION DE RESEAUX DE NEURONES

### Quelques éléments sur les réseaux de neurones en général

Un réseau de neurones est défini par son architecture, la règle d'apprentissage qui modifie les poids de liaisons entre neurones, et les fonctions de transfert de l'information d'une couche à l'autre. L'architecture comprend le nombre de couches, le nombre de neurones sur chaque couche et les liaisons entre les neurones des différentes couches. Le nombre de neurones de la première couche est la dimension de l'espace. Le réseau est dit supervisé si la sortie souhaitée de chaque neurone de la couche cachée est connue (voir par exemple J. Hérault & C. Jutten, "Réseaux neuronaux et traitement de signal", HERMES (1993)).
La figure 3 représente un réseau simple à trois couches.
Dans le cas de l'invention, les réseaux de neurones les plus adaptés sont les réseaux supervisés. Le nombre de neurones de la couche d'entrée est le nombre de variables (capteurs) décrivant les échantillons et celui de la couche de sortie est le nombre de classes (produits).
Les fonctions de transferts vers les neurones de sorties sont des sigmoïdes de tel sorte que la valeur attribué à chacun de ces neurones soit entre 0 et 1.
La réunion *VD* des ensembles *VDj* de variables synthétiques permettent en général une meilleure séparation des différents produits. Par conséquent, il est préférable de les utiliser pour construire des réseaux de neurones. En effet, parmi les variables initiales certaines sont redondantes et d'autres entachées de bruit. Tel n'est pas le cas des variables synthétiques.
Dans le premier mode de réalisation préféré de l'invention, ces variables sont utilisées pour construire un réseau de neurones global d'un type nouveau.

### Réseau de neurones global

Dans ce réseau, le nombre de neurones de la couche d'entrée est la dimension du sous espace *SED.* Chaque neurone est associé à une des variables synthétiques de l'ensemble *VD.* Le signal d'entrée de ce neurone est la valeur prise par cette variable sur le vecteur d'entrée. Le nombre de neurones de la couche de sortie est le nombre de produits.
Le nombre de couches cachées ainsi que celui de leur neurones respectifs peuvent être variables.
Le schéma de la figure 4 représente la procédure de construction de ce réseau.
Cette utilisation permet une convergence plus rapide que celle de l'utilisation classique car les variables utilisées sont les plus discriminantes et leur nombre est faible devant celui des capteurs. Dans la plupart des applications effectuées jusqu'à présent ce nombre est inférieur à cinq.

Une fois le réseau neuronal établi, l'appareil de classification peut être employé pour identifier des échantillons de produits inconnus, éventuellement après une validation du modèle d'identification inhérent au réseau neuronal développé. Une telle validation peut être effectuée en utilisant pour l'établissement du réseau neuronal les résultats de prétraitements statistiques effectuées sur la base des données relatives à un certain pourcentage seulement (par exemple 50%) des échantillons testés pendant la phase d'apprentissage, et en vérifiant si l'appareil réussit à classer correctement les données obtenues pour les autres échantillons.

Le deuxième mode de réalisation préféré de l'invention sera maintenant décrit à l'aide des figures 5 et 6. Le deuxième mode de réalisation peut utiliser un appareil du type dit "nez électronique" identique à celui utilisé dans le premier mode de réalisation. De plus, dans le deuxième mode de réalisation, au cours de la phase d'apprentissage, des données brutes sont acquises et soumises aux mêmes prétraitements statistiques que dans le premier mode de réalisation. Cependant, dans le deuxième mode de réalisation de l'invention, les résultats des prétraitements statistiques sont utilisés pour établir la structure d'un réseau neuronal dont la couche intermédiaire comporte une pluralité de réseaux neuronaux individuels. Chacun de ces réseau neuronaux individuels est associé à un produit. Pour chaque produit *PCj,* seuls les éléments de *VDj* sont pris en compte. Le réseau neuronal combinatoire possédant cette structure permet de réduire de façon significative le temps nécessaire pour la phase d'apprentissage. Ceci s'avère d'un intérêt particulier lorsque le nombre de produits à analyser est grand.

**Réseau individuel**

Dans la phase de prétraitement, un sous espace *SEj* est associé au produit *PCj.* Ce sous espace peut être le même pour plus d'un produit. Dans *SEj* le nuage de points est structuré en plusieurs régions dont une est associée au produit *PCj.* Chacune des autres régions est associée à un ou plusieurs produits. Ce cas de figure se présente lorsque certains des composants de certains produits sont les mêmes. La figure 5 illustre ce cas de figure. Les produits *PC1* et *PC2* sont bien séparés de tous les autres. Ce plan est donc le sous espace *SE1* et *SE2.*

Pour les produits *PCj* auxquels est associé un sous espace *SEj,* on construit un réseau qui les identifie. Le nombre pj de neurones de la couche d'entrée est la dimension du sous espace *SEDj.* Chaque neurone est associé à une des variables synthétiques de l'ensemble *VDj.* Le nombre de neurones de la couche de sortie est le nombre de régions bien discriminées sur *SEj.* Un certain nombre de produits sont donc identifiés par ce réseau.

Le nombre de couches cachées ainsi que celui de leur neurones respectifs peuvent être variables.

Le nombre des réseaux individuels est inférieur au nombre de produits analysés.

**Réseau combinatoire**

Ce réseau est en faite la combinaison de tous les réseaux individuels. La première couche de ce réseau est la même que celle du réseau global décrit ci-dessus en relation avec le premier mode de réalisation de l'invention. La deuxième couche est formée de l'ensemble des réseaux individuels décrits ci-dessus. Le nombre de neurones de la couche de sortie est le nombre de produits analysés. Chaque neurone représente un produit. Il est relié au plus à un neurone de sortie de chaque réseau individuel. Le signal de sortie de chacun de ces neurones est le maximum de ces entrées.

Pour entraîner ce réseau, on entraîne séparément les différents réseaux individuels.

Le schéma de la figure 6 illustre l'architecture d'un tel réseau.

Des modifications peuvent être apportées aux modes de réalisation décrits ci-dessus sans sortir du cadre de la présente invention. Par exemple, dans le cadre de l'application de l'invention à un appareil de type "nez électronique", dans les prétraitements statistiques, les données provenant des capteurs peuvent être associées à d'autres mesures provenant d'un ou plusieurs détecteurs de paramètres environnementaux (tels qu'un capteur de température ou un capteur d'humidité). Ces dernières mesures peuvent jouer un rôle particulier en définissant, par exemple, des métriques appropriées. D'autre part, dans la plupart des cas, les variables VDj induisent une discrimination linéaire du produit PCj des autres produits. Alors, au lieu de construire un réseau combinatoire comportant des réseaux individuels non-linéaires, on peut utiliser des réseaux individuels linéaires. Dans chacun de ces réseaux, on cherche les hyperplans qui séparent au mieux un ou plusieurs produits PCj. Un exemple de ces hyperplans est illustré à la figure 6 où les hyperplans sont indiqués en pointillés.

Bien que des modes de réalisation préférés de la présente invention aient été présentés ci-dessus, il convient de rappeler que la présente invention n'est pas limitée à ces modes de réalisation, décrits en tant qu'exemples non limitatifs.

**Revendications**

1. Appareil de classification, notamment destiné à une utilisation dans la reconnaissance ou la caractérisation d'odeurs, comprenant:

   un moyen destiné à acquérir, par une pluralité de canaux au cours d'une phase d'apprentissage, des données brutes représentant une pluralité d'instances d'une pluralité de classes différentes ;
   une unité de traitement destinée à traiter lesdites données brutes afin de déterminer pour chaque classe (j) des caractéristiques communes aux différentes instances de cette classe ; et
   un réseau neuronal destiné à émettre en sortie une classification dans une des classes d'une instance présentée audit appareil de classification, ledit réseau neuronal comportant une couche d'entrée, une couche de sortie et au moins une couche intermédiaire disposée entre les couches d'entrée et de sortie, la couche de sortie comprenant une pluralité de neurones chacun étant adapté à reconnaître des instances d'une classe respective de la pluralité des classes ;

   **caractérisé en ce que** l'unité de traitement de détermine pour chaque classe (j) un sous-espace (SEj) dans lequel des instances de cette classe (j), pour lesquelles des données brutes ont été obtenues, sont séparées de façon optimale des instances de toutes les autres classes, ledit sous-espace (SEj) étant défini par un ensemble (VDj) d'une ou plusieurs variables synthétiques (V), et de déterminer le sous-espace de discrimination (SED) qui comprend les sous-espaces (SEj) de toutes les classes; ledit sous-espace (SED) étant défini par la pluralité de variables (VD) comprises dans les ensembles (VDj) de variables synthétiques de la pluralité de classes ;
   et que la couche d'entrée du réseau neuronal comporte une pluralité de neurones chacun correspondant à une variable respective (V) de la pluralité de variables synthétiques (VD) qui définissent le sous-espace de discrimination (SED).

2. Appareil de classification selon la revendication 1, **caractérisé en ce que** l'unité de traitement sélectionne pour chaque classe (j) le sous-espace (SEj) dans lequel le groupement des instances de cette classe (j) est le plus éloigné des groupements des instances des autres classes et l'étendu du groupement de la classe (j) est minimisé.

3. Appareil de classification selon la revendication 1, **caractérisé en ce que**, dans un cas où dans tous les sous-espaces possibles au moins un groupement des instances d'une classe chevauche avec un groupement des instances d'une autre classe, l'unité de traitement sélectionne pour chaque classe (j) le sous-espace (SEj) dans lequel le rapport entre l'étendu du groupement des instances de cette classe (j) et le degré de chevauchement est maximal.

4. Appareil de classification selon la revendication 1, 2 ou 3, **caractérisé en ce que** l'unité de traitement sélectionne le sous-espace (SEj) à associer avec une classe (j) en déterminant, dans chacun d'une pluralité de sous-espaces différents, l'enveloppe convexe de la région (Rj) qui contient les points correspondants aux instances de ladite classe (j) et en déterminant le sous-espace dans lequel cette région (Rj) est le mieux séparée des régions correspondantes aux autres classes.

5. Appareil de classification selon la revendication 4, **caractérisé en ce que** l'unité de traitement identifie des instances d'une classe (j) comme étant aberrantes si le changement des dimensions de la région (Rj) nécessaire pour englober le point correspondant à cette instance représente une augmentation, supérieure à un certain pourcentage, des dimensions de la région (Rj) englobant les autres points relatifs à cette classe.

6. Appareil de classification selon l'une quelconque des revendications précédentes, **caractérisé en ce que** l'unité de traitement des données brutes est adaptée à identifier les canaux dont les données n'aident pas à la différenciation des instances de classes différentes, et à déterminer les sous-espaces (SEj), les ensembles (VDj) de variables synthétiques et le sous-espace de discrimination (SED) à partir de données qui excluent les données provenant du canal ou des canaux ainsi identifiés.

7. Appareil de classification selon l'une quelconque des revendications précédentes, **caractérisé en ce que** le réseau neuronal est un réseau supervisé dont les neurones appliquent des fonctions sigmoïdes et qui est entraîné en employant l'algorithme de propagation vers l'avant.

8. Appareil de classification selon l'une quelconque des revendications précédentes, **caractérisé en ce que** la couche, ou bien, les couches intermédiaire(s) du réseau neuronal comporte une pluralité de réseaux neuronaux individuels, l'un pour chacune de la pluralité de classes, les neurones de la couche d'entrée de chaque réseau individuel étant reliés au neurone, ou bien aux neurones, de la couche d'entrée du réseau global qui correspondent aux variables synthétiques (V) de l'ensemble (VDj) qui définissent le sous-espace (SEj) dans lequel les instances de cette classe sont différenciées des instances des autres classes.

9. Appareil de classification selon la revendication 8, **caractérisé en ce que** l'entraînement du réseau neuronal global comprend l'entraînement, séparé, des réseaux neuronaux individuels.

10. Appareil de classification selon l'une quelconque des revendications précédentes, **caractérisé en ce que** l'unité de traitement des données ne détermine les sous-espaces (SEj), les ensembles (VDj) de variables synthétiques et le sous-espace de discrimination (SED) qu'à partir de données brutes relatives à un certain pourcentage des instances pour lesquelles des données ont été acquises pendant la phase d'apprentissage, les données brutes concernant les autres instances étant utilisées pour effectuer une validation de l'efficacité de classification du réseau neuronal.

11. Appareil de classification selon l'une quelconque des revendications précédentes, **caractérisé en ce que** l'unité de traitement de données applique une analyse en composantes principales ou une analyse factorielle discriminante pour déterminer les variables synthétiques.

12. Procédé d'apprentissage d'un appareil de classification comprenant un moyen destiné à acquérir, par une pluralité de canaux, des données brutes représentant des instances d'une pluralité de classes différentes, une unité de traitement de ces données brutes et un réseau neuronal comportant une couche d'entrée, une couche de sortie et au moins une couche intermédiaire disposée entre les couches d'entrée et de sortie, la couche de sortie comprenant une pluralité de neurones chacun étant adapté à reconnaître des instances d'une classe respective de la pluralité des classes, cet appareil étant notamment destiné à une utilisation dans la reconnaissance ou la caractérisation d'odeurs, le procédé comportant les étapes consistant à:

soumettre au moyen d'acquisition de données brutes, pendant une phase d'apprentissage, une pluralité d'instances de la pluralité de classes, l'appartenance des instances aux classes étant connue ; et

traiter lesdites données brutes, par l'unité de traitement de données afin de déterminer pour chaque classe (j), des caractéristiques communes aux différentes instances de la classe ;

**caractérisé en ce que** l'étape de traitement consiste à traiter lesdites données brutes, par l'unité de traitement de données afin de déterminer pour chaque classe (j) un sous-espace (SEj) dans lequel des instances de cette classe (j), pour lesquelles des données brutes ont été obtenues, sont séparées de façon optimale des instances de toutes les autres classes, ledit sous-espace (SEj) étant défini par un ensemble (VDj) d'une ou plusieurs variables synthétiques (V), et de déterminer le sous-espace de discrimination (SED) qui comprend les sous-espaces (SEj) de toutes les classes, ledit sous-espace (SED) étant défini par la pluralité de variables (VD) comprises dans les ensembles (VDj) de variables synthétiques de la pluralité de classes ;

et que le procédé comporte en outre l'étape de munir le réseau neuronal d'une couche comportant une pluralité de neurones destinés à recevoir, respectivement, en entrée la valeur d'une variable respective de la pluralité de variables synthétiques (VD) qui définissent le sous-espace de discrimination (SED).

**Patentansprüche**

1. Klassifiziervorrichtung, insbesondere zur Verwendung beim Erkennen oder bei der Charakterisierung von Gerüchen, umfassend:

eine Einrichtung, die dazu ausgebildet ist, über eine Mehrzahl von Kanälen im Verlauf einer Lernphase Rohdaten zu erfassen, welche eine Mehrzahl von Objekten einer Mehrzahl verschiedener Klassen repräsentieren;

eine Behandlungseinheit, ausgebildet zur Behandlung der genannten Rohdaten, um für jede Klasse (j) gemeinsame Charakteristika für verschiedene Objekte dieser Klasse zu bestimmen; und

ein neuronales Netzwerk, dazu ausgebildet, am Ausgang eine Klassifikation in eine der Klassen von einem Objekt auszugeben, das der Klassifiziervorrichtung eingangsseitig angeboten wird, wobei das neuronale Netzwerk eine Eingangsschicht, eine Ausgangsschicht und mindestens eine Zwischenschicht zwischen der Eingangsschicht und der Ausgangsschicht aufweist, von denen die Ausgangsschicht eine Mehrzahl von Neuronen beinhaltet, jeweils ausgebildet zum Erkennen von Objekten aus einer der mehreren Klassen;

**dadurch gekennzeichnet, dass** die Behandlungseinheit für jede Klasse (j) einen Unterraum (SEj) bestimmt, in welchem die Objekte dieser Klasse (j), für welche die Rohdaten erhalten wurden, in optimaler Weise getrennt sind von Objekten sämtlicher anderer Klassen, wobei der Unterraum (SEj) definiert ist durch eine Menge (VDj) mit einer oder mehreren synthetischen Variablen (V), und den Diskriminations-Unterraum (SED) bestimmt, der die Unterräume (SEj) sämtlicher Klassen enthält, wobei der Unterraum (SED) definiert wird durch die mehreren Variablen (VD), die in den Mengen (VDj) von synthetischen Variablen der mehreren Klassen enthalten sind;

und dass die Eingangsschicht des neuronalen Netzwerks eine Mehrzahl von Neuronen aufweist, die jeweils einer zugehörigen Variablen (V) der mehreren synthetischen Variablen (VD) entsprechen, welche den Diskriminations-Unterraum (SED) definieren.

2. Klassifiziervorrichtung nach Anspruch 1,
**dadurch gekennzeichnet, dass** die Behandlungseinheit für jede Klasse (j) den Unterraum (SEj) aussucht, in welchem die Gruppierung der Objekte dieser Klasse (j) am weitesten entfernt ist von den Gruppierungen von Objekten der übrigen Klassen und die Erstreckung der Gruppierung der Klasse (j) minimiert ist.

3. Klassifiziervorrichtung nach Anspruch 1,
**dadurch gekennzeichnet, dass** in einem Fall, in welchem in sämtlichen möglichen Unterräumen mindestens eine Gruppierung der Objekte einer Klasse sich mit einer Gruppierung von Objekten einer anderen Klasse überlappt, die Behandlungseinheit für jede Klasse (j) den Unterraum (SEj) auswählt, in welchem die Beziehung der Erstreckung der Gruppierung von Objekten dieser Klasse (j) und dem Grad der Überlappung maximal ist.

4. Klassifiziervorrichtung nach Anspruch 1,2 oder 3,
**dadurch gekennzeichnet, dass** die Behandlungseinheit den einer Klasse zuzuordnenden Unterraum (SEj) dadurch auswählt, dass sie in jedem aus einer Mehrzahl verschiedener Unterräume die konvexe Umhüllende der Zone (Rj) bestimmt, die diejenigen Punkte enthält, die den Objekten dieser Klasse (j) entsprechen und den Unterraum bestimmt, in welchem diese Zone (Rj) am besten von entsprechenden Zonen anderer Klassen getrennt ist.

5. Klassifiziervorrichtung nach Anspruch 4,
**dadurch gekennzeichnet, dass** die Behandlungseinheit Objekte einer Klasse (j) als Ausreißer identifiziert, wenn die Änderung der Abmessungen der Zone (Rj), die notwendig ist, um den diesem Objekt entsprechenden Punkt einzufassen, eine Vergrößerung darstellt, die über einem gewissen Prozentsatz der Abmessungen derjenigen Zone (Rj) liegt, welche die übrigen Punkte bezüglich dieser Klasse umschließt.

6. Klassifiziervorrichtung nach einem der vorhergehenden Ansprüche,
**dadurch gekennzeichnet, dass** die Behandlungseinheit der Rohdaten dazu ausgebildet ist, die Kanäle zu identifizieren, deren Daten keinen Beitrag leisten zu der Differenzierung von Objekten der verschiedenen Klassen, und die Unterräume (SEj), die Mengen (VDj) synthetischer Variablen und den Diskriminations-Unterraum (SED) an Hand von Daten zu bestimmen, welche aus dem identifizierten Kanal oder den identifizierten Kanälen, stammen.

7. Klassifiziervorrichtung nach einem der vorhergehenden Ansprüche,
**dadurch gekennzeichnet, dass** das neuronale Netzwerk ein überwachtes Netzwerk ist, dessen Neuronen Sigmoid-Funktionen anwenden, und das unter Verwendung des Algorithmus der Vorwärtsfortpflanzung geschult wird.

8. Klassifiziervorrichtung nach einem der vorhergehenden Ansprüche,
**dadurch gekennzeichnet, dass** die Zwischenschicht oder Zwischenschichten des neuronalen Netzwerks eine Mehrzahl von individuellen neuronalen Netzwerken beinhaltet bzw. beinhalten, eines für jede der mehreren Klassen, wobei die Neuronen der Eingangsschicht jedes individuellen Netzwerks mit dem Neuron bzw. den Neuronen der Eingangsschicht des globalen Netzwerks verbunden ist/sind, welches den synthetischen Variablen (V) der Menge (VDj) entsprechen, die den Unterraum (SEj) definieren, in welchem die Objekte dieser Klasse unterschieden sind von den Objekten anderer Klassen.

9. Klassifiziervorrichtung nach Anspruch 1,
**dadurch gekennzeichnet, dass** das Schulen des globalen neuronalen Netzwerks die getrennte Schulung der individuellen neuronalen Netzwerke beinhaltet.

**10.** Klassifiziervorrichtung nach einem der vorhergehenden Ansprüche,
**dadurch gekennzeichnet, dass** die Behandlungseinheit für Daten die Unterräume (SEj), die Mengen (VDj) synthetischer Variablen und den Diskriminations-Unterraum (SED) ausschließlich an Hand von Rohdaten bestimmt, die sich auf einen gewissen Prozentsatz von Objekten beziehen, für die die Daten während der Lernphase erfasst wurden, wobei die Rohdaten bezüglich anderer Objekte dazu verwendet werden, eine Wirksamkeit der Klassifikation des neuronalen Netzwerks nachzuweisen.

**11.** Klassifiziervorrichtung nach einem der vorhergehenden Ansprüche,
**dadurch gekennzeichnet, dass** die Behandlungseinheit der Daten zur Bestimmung der synthetischen Variablen eine Hauptkomponentenanalyse oder eine Diskriminanten-Faktorenanalyse durchführt.

**12.** Verfahren zum Schulen einer Klassifiziervorrichtung, die eine Einrichtung enthält, die dazu ausgebildet ist, über eine Mehrzahl von Kanälen Rohdaten zu erfassen, die Objekte aus einer Mehrzahl unterschiedlicher Klassen repräsentieren, eine Behandlungseinheit für diese Rohdaten und ein neuronales Netzwerk aufweist, welches eine Eingangsschicht, eine Ausgangsschicht und mindestens eine zwischen der Eingangsschicht und der Ausgangsschicht befindliche Zwischenschicht enthält, von denen die Ausgangsschicht eine Mehrzahl von Neuronen enthält, die jeweils dazu ausgebildet sind, Objekte einer Klasse von den mehreren Klassen wiederzuerkennen, wobei die Vorrichtung insbesondere zur Verwendung beim Erkennen oder bei der Charakterisierung von Gerüchen bestimmt ist, wobei das Verfahren folgende Schritte umfasst:

Während einer Lernphase wird die Rohdaten-Erfassungseinrichtung einer Mehrzahl von Objekten der mehreren Klassen ausgesetzt, wobei die Zugehörigkeit der Objekte zu den Klassen bekannt ist; und

die Rohdaten werden von der Behandlungseinheit für Daten mit dem Zweck behandelt, für jede Klasse (j) Charakteristika zu bestimmen, die unterschiedlichen Objekten der Klasse gemeinsam sind;

**dadurch gekennzeichnet, dass** der Behandlungsschritt darin besteht, dass die Rohdaten von der Datenbehandlungseinheit mit dem Zweck behandelt werden, für jede Klasse (j) einen Unterraum (SEj) zu bestimmen, in welchem Objekte dieser Klasse (j), für die die Rohdaten erhalten wurden, in optimaler Weise getrennt sind von Objekten sämtlicher übriger Klassen, wobei der Unterraum (SEj) definiert wird durch eine Menge (VDj) mit einer oder mehreren synthetischen Variablen (V), und den Diskriminations-Unterraum (SED) zu bestimmen, der die Unterräume (SEj) sämtlicher Klassen enthält, wobei der Unterraum (SED) definiert ist durch die mehreren Variablen (VD) in den Mengen (VDj) synthetischer Variabler der mehreren Klassen;
und dass das Verfahren außerdem den Schritt beinhaltet, das neuronale Netzwerk mit einer Schicht auszustatten, welche mehrere Neuronen enthält, die dazu bestimmt sind, eingangseitig den Wert einer Variablen aus den mehreren synthetischen Variablen (VD) zu empfangen, welche den Diskriminations-Unterraum (SED) definieren.

## Claims

**1.** Classification apparatus, notably for use in the recognition or the characterisation of odours, comprising:

means for acquiring, by a plurality of channels during a learning phase, raw data representing a plurality of instances of a plurality of different classes;
a processing unit for processing said raw data so as to determine, for each class (j), characteristics common to the different instances of this class; and
a neural network for outputting a classification, into one of the classes, of an instance presented to said classification apparatus, said neural network comprising an input layer, an output layer and at least one intermediate layer disposed between the input and output layers, the output layer comprising a plurality of neurones each being adapted to recognise instances of a respective class from the plurality of classes;

**characterised in that** the processing unit determines for each class (j) a sub-space (SEj) in which the instances of this class (j), for which raw data has been obtained, are optimally separated from the instances of all the other classes, said sub-space (SEj) being defined by a set (VDj) of one or a plurality of synthetic variables (V), and determining the discrimination sub-space (SED) which comprises the sub-spaces (SEj) of all of the classes, said sub-space (SED) being defined by the plurality of variables (VD) comprised in the sets (VDj) of synthetic variables of the plurality of classes;
and **in that** the input layer of the neural network comprises a plurality of neurones each corresponding to a respective variable (V) of the plurality of synthetic variables (VD) which define the discrimination sub-space (SED).

2. Classification apparatus according to claim 1, **characterised in that** the processing unit selects for each class (j) the sub-space (SEj) in which the cluster of instances of this class (j) is the farthest from the clusters of instances of the other classes and the spread of the cluster of the class (j) is minimised.

3. Classification apparatus according to claim 1, **characterised in that**, in a case where in all the possible sub-spaces at least one cluster of the instances of a class overlaps with a cluster of the instances of another class, the processing unit selects for each class (j) the sub-space (SEj) in which the ratio of the spread of the cluster of instances of this class (j) to the degree of overlap is maximised.

4. Classification apparatus according to claim 1, 2 or 3, **characterised in that** the processing unit selects the sub-space (SEj) to associate with a class (j) by determining, in each of a plurality of different sub-spaces, the convex envelope of the region (Rj) which contains the points corresponding to the instances of said class (j) and by determining the sub-space in which this region (Rj) is best separated from the regions corresponding to the other classes.

5. Classification apparatus according to claim 4, **characterised in that** the processing unit identifies instances of a class as being abnormal if the change in the dimensions of the region (Rj) necessary to encompass the point corresponding to this instance represents an increase, greater than a certain percentage, of the dimensions of the region (Rj) that encircles the other points relating to this class.

6. Classification apparatus according to any one of the preceding claims, **characterised in that** the raw data processing unit is adapted to identify the channels whose data does not help to differentiate instances of different classes, and to determine the sub-spaces (SEj), the sets (VDj) of synthetic variables and the discrimination sub-space (SED) based on data excluding the data from the thus-identified channel(s).

7. Classification apparatus according to any one of the preceding claims, **characterised in that** the neural network is a supervised network the neurones of which apply sigmoid functions and which is trained using the back propagation algorithm.

8. Classification apparatus according to any one of the preceding claims, **characterised in that** the intermediate layer, or layers, of the neural network comprise a plurality of individual neural networks, one for each of the plurality of classes, the neurones of the input layer of each individual network being connected to the neurone, or neurones, of the input layer of the global network which correspond to the synthetic variables of the set (VDj) which defines the sub-space (SEj) in which the instances of this class are differentiated from the instances of the other classes.

9. Classification apparatus according to claim 8, **characterised in that** the training of the complete neural network comprises the separate training of the individual neural networks.

10. Classification apparatus according to any one of the preceding claims, **characterised in that** the data processing unit determines the sub-spaces (SEj), the sets (VDj) of synthetic variables and the discrimination sub-space (SED) based only on raw data relating to a certain percentage of the instances for which data has been acquired during the learning phase, the raw data relating to the other instances being used to perform a validation of the classification effectiveness of the neural network.

11. Classification apparatus according to any one of the preceding claims, **characterised in that** the data processing unit applies a principal component analysis or a discriminant factorial analysis to determine the synthetic variables.

12. A learning method of a classification apparatus comprising means for acquiring, by a plurality of channels, raw data representing instances of a plurality of different classes, a unit processing said raw data and a neural network comprising an input layer, an output layer and at least one intermediate layer disposed between the input and output layers, the output layer comprising a plurality of neurones each being adapted to recognise instances of a respective class from the plurality of classes, this apparatus being notably for use in the recognition or characterisation of odours, the method comprising the steps of:

applying to the raw data acquisition means, during a learning phase, a plurality of instances of the plurality of classes, the classes to which the instances belong being known; and

processing said raw data, by the data processing unit, so as to determine for each class (j) characteristics common to the different instances of the class;

**characterised in that** the processing step consists in processing said raw data, by the data processing unit, so as to determine, for each class (j), a sub-space (SEj) in which the instances of this class (j), for which raw data has been obtained, are optimally separated from the instances of all the other classes, said sub-space (SEj) being defined by a set (VDj) of one or a plurality of synthetic variables (V), and in determining the discrimination sub-space (SED) which comprises the sub-spaces (SEj) of all the classes, said sub-space (SED) being defined by the plurality of variables (VD) comprised in the sets (VDj) of synthetic variables of the plurality of classes;

and **in that** the method further comprises the step of providing the neural network with a layer comprising a plurality of neurones for receiving, respectively, as input the value of a respective variable of the plurality of synthetic variables (VD) defining the discrimination sub-space (SED).

Figure 1 : Recherche des régions spécifiques

**Figure 2A : Plan Discriminant**

**Figure 2B : enveloppes convexes délimitant les différentes régions**

Couche d'entrée     Couche cachée     Couche de sortie

X1   ( E )

X2   ( E )

X3   ( E )

X4   ( E )

X5   ( E )

X6   ( E )

( H )

( H )

( H )

( O )

( O )

**Figure 3 : Réseau de neurones**

Variables Brutes
Mesures des capteurs

Variables Synthétiques
les discriminantes

Prétraitement statistique
Définition des régions Rj

Réseau de
neurones

**Figure 4 : Schéma de construction du réseau de neurones**

Figure 5 : Exemple de sous espace Sej

Figure 6 : Architecture du réseau combinatoire